# EUROPEAN PATENT SPECIFICATION

(11) **EP 3 288 397 B1**
(45) Date of publication and mention of the grant of the patent: **26.06.2019**
(21) Application number: 16721737.1
(22) Date of filing: 26.04.2016
(51) Int. Cl.: A23L 5/44, A23L 2/58, A23L 5/40

(54) **WATER-DISPERSIBLE COLORING COMPOSITION**
WASSERDISPERGIERBARE FÄRBUNGSZUSAMMENSETZUNG
COMPOSITION DE COLORANT DISPERSIBLE DANS L'EAU

(30) Priority: 28.04.2015 EP 15165332; 09.02.2016 EP 16154833
(43) Date of publication of application: 07.03.2018
(73) Proprietor: Chr. Hansen Natural Colors A/S, 2970 Hørsholm (DK)
(72) Inventor: KOEHLER, Klaus, 1820 Frederiksberg (DK); NAUD, Juliette, 2000 Frederiksberg (DK)
(86) International application number: PCT/EP2016/059232
(87) International publication number: WO 2016/174004

(56) References cited:
- WO-A1-2008/032006
- WO-A1-2014/033703
- US-A1- 2003 129 290
- US-A1- 2011 177 202
- US-A1- 2013 337 024
- US-B1- 6 287 615

## Description

### FIELD OF INVENTION

The present invention relates to the field of coloring agents comprising carotenoid natural pigments in the form of solid particles being dispersed in an aqueous phase comprising polysorbate as a water-soluble emulsifier, use of the coloring agents, edible products comprising the coloring agents and a method for preparing the coloring agents.

### BACKGROUND OF THE INVENTION

Coloring agents containing natural or synthetic coloring substances are commonly used as additives in the manufacturing of food products and pharmaceutical products. A wide range of such coloring agents are commercially available making it possible for the manufacturer, where a particular color tone is desired, to select a single coloring agent having the desired color or a mixture of coloring agents, which in an appropriate combination impart the desired color to the product. Or alternatively, the manufacturer may wish to use an agent which, in addition to its coloring effect, has a health improving effect and hence select such a single health improving agent or a mixture of such agents.

The commercially available coloring agents can contain synthetic substances including substances that are also normally referred to as dyes or azodyes, or such agents can contain pigments or other coloring substances of natural origin, e.g. in the form of plant material containing a coloring substance, or as more or less purified coloring substances separated from plant, animal or microbial materials naturally containing such substances.

Occasionally, food grade or pharmaceutically acceptable colorings agents are provided that contain coloring substances in the form of synthetic or artificial compounds having substantially the same chemical composition as natural occurring coloring agents. These types of coloring agents are also referred to in the art as "nature identical" colors.
In the present context, the expressions "naturally occurring coloring agents" and "natural pigments" also include "nature identical" colors.

Natural pigments or nature-identical pigments may be water soluble or water insoluble. The present invention relates to water insoluble pigments.

Any of the above types of available coloring agents including agents comprising food grade or pharmaceutically acceptable natural coloring substances can be water soluble, sparingly soluble in water and aqueous media or essentially water-insoluble. A water soluble coloring material or substance as such requires that the product to be colored has an aqueous phase in which the coloring substance is soluble.

However, it may be desirable to obtain the color tone of a particular coloring substance that is insoluble or sparingly soluble in an aqueous phase or a mixture of such substances in a food product or a pharmaceutical product that does not comprise a phase in which the coloring substance is sufficiently soluble to provide the desired coloring. There is therefore a continuous industrial need for coloring agents containing coloring substances that are water insoluble or sparingly insoluble in water, which are in the form of water-miscible or water dispersible compositions having a high coloring efficiency.

Carotenoids which have yellow, orange or red colors occur widely in nature and important sources are plants including grasses, the annatto tree, citrus species, *Capsicum annum*, *Crocus sativus* flowers and marigold flowers, marine algae, yeast and some animals. Carotenoids can be divided into the following classes: carotenoid hydrocarbons, xanthophylls and apocarotenoids. Typical examples of carotenoids include bixin, *β-*carotene, apocarotenals, canthaxanthin, saffron, crocin, capsanthin and capsorubin occurring in paprika oleoresin, lutein, astaxanthin, rubixanthin, violaxanthin, rhodoxanthin, lycopene and derivatives hereof.

Carotenoids are generally understood to be essentially water-insoluble or sparingly soluble in water.

The working Examples of US2011/177202A1 application describe coloring compositions comprising paprika oleoresin (carotenoid natural pigment) and the emulsifiers, polysorbate and "citric acid ester of mono- and diglycerides of edible fatty acids (E472c)". As known in the art and also explained in paragraph [0020] of the US application - paprika oleoresin is an oil-soluble extract of paprika that contains carotenoids such as capsanthin, capsorubin and β-carotene - accordingly, paprika oleoresin is oil with dissolved carotenoids.

Accordingly, the coloring compositions made in the working examples of US2011/177202A1 may be described as oil-in-water (o/w) emulsion color compositions based on oil with dissolved carotenoids (paprika oleoresin) - i.e. liquid oil droplets (comprising dissolved carotenoids) dispersed in water.
According to paragraph [0045] the paprika oleoresin is mixed with emulsifying agents and agitated until completely dissolved to get a homogenous oil phase and an aqueous solution is added to the oil phase to get the oil-in-water (o/w) emulsion, which is homogenized in order to get smaller liquid oil droplets (comprising dissolved carotenoids) dispersed in water.
As understood by the skilled person in the present context - such "liquid oil droplets" are not understood to be "solid particles".

As known in the art - the term "emulsion" is used when both phases, dispersed and continuous, are liquids - i.e. like for example above discussed paprika oleoresin oil-in-water (o/w) emulsion compositions.
A solid-in-water dispersion, wherein the solid particles (e.g. solid beta-carotene particles) are dispersed in the water phase of the solid-in-water dispersion is not understood to be an emulsion.

Accordingly, a paprika oleoresin based oil-in-water (o/w) emulsion as described in the working examples of US2011/177202A1 is not a water-dispersible composition comprising a dispersion of at least 2% (w/w) of carotenoid as the natural pigment in the form of solid particles as discussed herein.

In the working examples of US2011/177202A1 were used at least 5 times more polysorbate than paprika oleoresin - i.e. the ratio (w/w) of polysorbate emulsifier: carotenoid pigment were at least 5:1. In line of this is there in paragraph [0033] explained that it is preferred to use at least 5 times more polysorbate than coloring pigment. For herein relevant commercial use, this may be seen as a too high amount of used polysorbate - one reason for this relates to that polysorbate may be said to have an unwanted unpleasant taste (see e.g. WO2013/167644A1).

Paragraph [0047] of US2011/177202A1 reads: "If only polysorbate 60 is used as the emulsifying agent, the solution consisting of the composition and the lemonade medium becomes macroscopically inhomogeneous" - accordingly use of polysorbate as such did not work properly at the low pH of the lemonade medium.

The working examples of WO2014/033703A1 application describe solid-in-water dispersions comprising β-carotene pigment solid particles dispersed in an aqueous phase comprising different not polysorbate emulsifiers - the term "polysorbate" in not mentioned at all in the WO application. The relevant working examples use sugar ester as emulsifier and such sugar ester may be considered relatively acid labile and therefore not very stable at low pH values.

CN102652732A discloses the preparation of nanoscale water-soluble emulsions, with a particle size of 100-600 nm, by homogenization of carotenoids in an aqueous medium also comprising emulsifiers such as tween (polysorbate) or polyglycerol ester of fatty acid, and a hydrocolloid. The active compound (e.g. a carotenoid) is dissolved in an organic solvent/emulsifier (see e.g. Example 3) and the pigment is therefore not in the form of solid particles.

US2013/337024A1 describes oil-in-water (o/w) emulsion color compositions based on oil with dissolved carotenoids (e.g. lycopene - see [0250]) - i.e. liquid oil droplets (comprising dissolved carotenoids) dispersed in water.

Since edible products, due to consumer taste preferences, extended shelf life etc., often have a low pH, the provision of new food grade coloring agents which are stable at low pH is highly desired.
Especially, water-dispersible coloring compositions/products based on water-insoluble carotenoid natural pigments, which do not precipitate at a pH below 4, are desired - e.g. to be used for coloring a soft drink.

### SUMMARY OF THE INVENTION

The problem to be solved by the present invention relates to the provision of a novel carotenoid (e.g. bixin or beta-carotene) water-dispersible coloring composition with a relatively high concentration of water-insoluble carotenoid pigment and which does not precipitate at a pH below 4 and therefore may e.g. be used for coloring a soft drink.

The solution is based on that the present inventors identified that polysorbate is a particular useful emulsifier in order to make a concentrated (i.e. with high coloring power) carotenoid pigment solid-in-water dispersion product, wherein the solid carotenoid pigment particles are stably dispersed as small solid particles (e.g. size of less than 1 µm) in the water phase and which do not precipitate at a pH below 4 and therefore may e.g. be used for coloring a soft drink.

The working examples 1 to 5 herein demonstrated that by use of polysorbate as emulsifier, it was possible to make concentrated (strength of at least 10% (w/w)) beta-carotene and bixin pigment solid-in-water dispersion compositions, which e.g. had following positive characteristics:
(i): *stable after storage for 3 months*; since the examples demonstrated that the small average particle size (less than 0.5 µm) of the solid pigment particles were stably maintained during 3 months storage;
(ii): *does not precipitate at a pH below 4*; since a low and stable average particle size was obtained at pH 3.0-3.5.

As known in the art, there is a close link between a low average particle size and a high coloring power (e.g. high color brightness (Chroma)).
Accordingly, the fact that a low and stable average particle size was obtained in the examples demonstrated that a high and stable coloring power was obtained at pH 3.0-3.5.

Working examples herein show that it is possible to obtain a good carotenoid color products even with use of relatively small amounts of polysorbate as emulsifier - for instance in working example 1 was only used half of the amount (w/w) of polysorbate relatively to the amount of beta-carotene - i.e. the ratio (w/w) of polysorbate emulsifier: carotenoid pigment is 1:2.
In beta-carotene working example 3 herein is the ratio (w/w) of polysorbate emulsifier: carotenoid pigment 1:1 (i.e. same amount (w/w) of polysorbate and beta-carotene).
In WO2014/033703A1 (discussed above) were used significantly more not polysorbate emulsifiers and the obtained solid-in-water dispersions therefore had a relatively lower color strength.

An advantage of the carotenoid water-dispersible composition as described herein is that they comprise a relatively high pigment concentration (such as e.g. least 5% (w/w)), which gives a more concentrated product. Advantages of a more concentrated product are shorter production process time per kg pigment and less expenses relating to e.g. transportation.

The low concentration of polysorbate water-soluble emulsifier needed in the coloring composition of the present invention to achieve acid stability as well as high tinctorial power assures that costs of production can be kept at a minimum and the inventors have found that no off-taste from the water soluble emulsifier is detectable in the finished colored edible product (e.g. a soft drink).

As shown in comparative working examples herein - polysorbate did not work properly for color pigments such as curcumin, carbo and chlorophyllin.
Without being limited to theory - it was a surprise for the present inventors that polysorbate works so well for bixin and β-carotene in view of that it did not work properly for other commercial relevant hydrophobic water-insoluble pigments such as curcumin, carbo and chlorophyllin.

As can be seen in comparative Example B herein - for instance "citric acid esters of fatty acid mono/diglycerides" is NOT working satisfactory for beta-carotene - i.e. one may say that polysorbate works surprisingly better.
Polysorbate and "citric acid esters of fatty acid mono/diglycerides" have both a relatively high Hydrophilic-lipophilic balance (HLB) value (i.e. are both considered to be water soluble emulsifiers) - however, even though "citric acid esters of fatty acid mono/diglycerides" has a relatively high HLB value it is not working properly in the present context.

An advantage of polysorbate as compared to e.g. emulsifiers such as polyglycerol and quillaja is that polysorbate is cheaper than e.g. polyglycerol and quillaja.
Further, in some countries (e.g. Europe and USA) there are some food regulatory legal restrictions in relation to e.g. polyglycerol and quillaja - to the contrary polysorbate is generally allowed to be used for food products in the majority of countries (e.g. Europe and USA).

As discussed above - working examples herein demonstrate that polysorbate works very well for bixin and β-carotene.

Without being limited to theory - in the present context, there is no prima facie reason to believe that polysorbate should not also work well for other similar hydrophobic carotenoid pigments such as apocarotenals, canthaxanthin, saffron, crocin, capsanthin, capsorubin, lutein, astaxanthin, rubixanthin, violaxanthin, rhodoxanthin or lycopene.

Accordingly, a first aspect of the present invention relates to a water-dispersible composition comprising a dispersion of at least 2% (w/w) of carotenoid natural hydrophobic pigment in the form of solid particles of an average size of at the most 10 µm, said solid particles being dispersed in an aqueous phase comprising polysorbate as a water-soluble emulsifier and
wherein the ratio (w/w) of polysorbate emulsifier:carotenoid pigment is in the range of 1:10 to 4:1; and
wherein the carotenoid natural hydrophobic pigment is at least one pigment selected from the group consisting of: bixin, β-carotene, α-carotene, apocarotenals, canthaxanthin, saffron, crocin, capsanthin, capsorubin, lutein, astaxanthin, rubixanthin, violaxanthin, rhodoxanthin and lycopene.

A second aspect of the invention relates to the use of a water-dispersible composition according to the first aspect and herein relevant embodiments thereof for the manufacture of an edible product or a pharmaceutical product.
A third aspect of the invention relates to an edible product or a pharmaceutical product comprising a composition according to the first aspect and herein relevant embodiments thereof.

A fourth aspect of the invention relates to a method for preparing a water-dispersible pigment composition of the first aspect and herein relevant embodiments thereof, the method comprising preparing a dispersion of at least 2% (w/w) of carotenoid natural hydrophobic pigment in the form of solid particles by comminuting the pigment in an aqueous phase comprising polysorbate as a water-soluble emulsifier, to obtain a dispersion comprising the pigment in the form of solid particles of an average size of at the most 10 µm.

### DETAILED DESCRIPTION OF THE INVENTION

The inventors of the present invention have found that a water-dispersible composition of the first aspect and herein relevant embodiments thereof provides a coloring agent with a very high tinctorial power and acid stability compared to conventional coloring agents currently on the market.

The water-dispersible composition according to the invention comprises at least one carotenoid natural pigment.

As discussed above - occasionally, food grade or pharmaceutically acceptable colorings agents are provided that contain coloring substances in the form of synthetic or artificial compounds having substantially the same chemical composition as natural occurring coloring agents. These types of coloring agents are also referred to in the art as "nature identical" colors.
In the present context, the expressions "naturally occurring coloring agents" and "natural pigments" also include "nature identical" colors.
As known in the art - β-carotene is an example of a pigment that may be obtained directly from a natural source and/or be a so-called nature identical color.

The natural pigment can be any food grade or pharmaceutically acceptable coloring matter derived from a natural source. Thus, the pigment may either be in a substantially pure form or it may be contained in the material where it occurs naturally such as a plant or animal material, optionally in combination with a food grade and/or pharmaceutically acceptable carrier.

It is essential that the pigments used in the current composition are solid water insoluble pigments.

Hydrophobic pigments are not soluble in water at any pH value.

Carotenoids which have yellow, orange or red colors occur widely in nature and important sources are plants including grasses, the annatto tree, citrus species, *Capsicum annum*, *Crocus sativus* flowers and marigold flowers, marine algae, yeast and some animals.

Carotenoids can be divided into the following classes: carotenoid hydrocarbons, xanthophylls and apocarotenoids.

As discussed above in relation to the first aspect - the carotenoid natural hydrophobic pigment is at least one pigment selected from the group consisting of: bixin, β-carotene, α-carotene, apocarotenals, canthaxanthin, saffron, crocin, capsanthin, capsorubin, lutein, astaxanthin, rubixanthin, violaxanthin, rhodoxanthin and lycopene.

The water-dispersible composition as described herein may comprise two or more different carotenoids - for instance 3% (w/w) of bixin and 6% (w/w) β-carotene, which give a composition with 9% (w/w) of carotenoid natural hydrophobic pigment.

Preferably, the carotenoid natural hydrophobic pigment is at least one pigment selected from the group consisting of: bixin and β-carotene.

The present invention relates to a water-dispersible composition comprising a dispersion of at least 2%, such as at least 3%, such as at least 4%, such as at least 5%, such as at least 6%, such as at least 7%, such as at least 8%, such as at least 9%, such as at least 10% (w/w) of carotenoid natural hydrophobic pigment as described above in the form of solid particles of an average size of at the most 10 µm or even less, said particles being dispersed in an aqueous phase comprising at least one water-soluble polysorbate emulsifier.

It may be preferred that the water-dispersible composition as described herein is comprising a dispersion of less than 50% (w/w), such as less than 40% (w/w) of carotenoid natural hydrophobic pigment.

In preferred embodiments, the average size of the solid particles is at the most 9 µm, such as at the most 8 µm, such as at the most 7 µm, such as at the most 6 µm, such as at the most 5 µm, such as the most 4 µm, such as at the most 3 µm, such as at the most 2 µm, such as at the most 1.9 µm, such as at the most 1.8 µm, such as at the most 1.7 µm, such as at the most 1.6 µm, such as at the most 1.5 µm, such as at the most 1.4 µm, such as the most 1.3 µm, such as at the most 1.2 µm, such as the most 1.1 µm, such as the most 1.0 µm, such as at the most 0.9 µm, such as at the most 0.8 µm, such as at the most 0.7 µm, such as at the most 0.6 µm, such as at the most 0.5 µm, such as at the most 0.4 µm, such as at the most 0.3 µm.

In relation to measurement of size of the particles - in working examples herein were used Malvern MasterSizer and there was measured D(4,3). As known in the art - D(4,3) relates to mean diameter by volume (i.e. the same as mass).
Accordingly and as understood by the skilled person in the present context - the average size of the particles relates to D(4,3) measured Malvern MasterSizer.

Polysorbates are a class of emulsifiers used in some pharmaceuticals and food preparation. Polysorbates are oily liquids derived from ethoxylated sorbitan (a derivative of sorbitol) esterified with fatty acids. Common brand names for polysorbates include Scattics, Alkest, Canarcel, and Tween.
As discussed above - polysorbates are very cost-efficient and this is a herein relevant advantage of the use of polysorbate as emulsifier as discussed herein.

The polysorbate may e.g. be polysorbate 20, polysorbate 40, polysorbate 60, polysorbate 65, polysorbate 80 or a mixture of these.
In working examples herein was used polysorbate 80 and it may be a preferred polysorbate.

Suitable examples of polysorbate include polysorbate with E-numbers: E 431, E 432, E 433, E 434, E 435 or E 436.
As known in the art - E-numbers are codes for substances that can be used as food additives for use within the European Union and the E-numbers are not changed over time and E-numbers therefore directly and unambiguously technically define a food additive of interest.

In a preferred embodiment the water-soluble emulsifier is present in the composition in an amount of at least 5% (w/w), such as at least 6% (w/w), such as at least 7% (w/w), such as at least 8% (w/w), such as at least 9% (w/w), such as at least 10% (w/w), such as at least 11% (w/w), such as at least 12% (w/w), such as at least 13% (w/w), such as at least 14% (w/w), such as at least 15% (w/w), and such as at least 20% (w/w).

In a preferred embodiment the water-soluble emulsifier is present in the composition in an amount of at the most 35% (w/w), such as at the most 25% (w/w), such as at the most 24% (w/w), such as at the most 23% (w/w), such as at the most 22% (w/w), such as at the most 21% (w/w), such as at the most 20% (w/w).

The low concentration of water-soluble emulsifier needed in the coloring composition of the present invention to achieve acid stability as well as high tinctorial power assures that costs of production can be kept at a minimum and the inventors have found that no off-taste from the water soluble emulsifier is detectable in the finished edible product (e.g. a soft drink).

As discussed above in relation to the first aspect - the ratio (w/w) of polysorbate emulsifier:carotenoid pigment is in the range of 1:10 to 4:1, more preferably in the range of 1:10 to 3:1.

As discussed above - working examples herein show that it is possible to obtain good carotenoid color products even with use of relatively small amounts of polysorbate as emulsifier, which is an advantage of the present invention.
Preferably there is not used more polysorbate than required in order to make a water-dispersible composition as described herein.

It may be preferred the ratio (w/w) of polysorbate emulsifier:carotenoid pigment is in the range of 1:4 to 4:1, such as 1:4 to 3:1 or such as 1:3 to 3:1. In a preferred embodiment of the invention the ratio (w/w) of polysorbate emulsifier: carotenoid pigment is in the range of 1:4 to 2:1, such as 1:2.5 to 2.5:1 or such as 1:2 to 2:1.

The water-dispersible composition as described herein may comprise other types of emulsifier than polysorbate.
However, it is preferred that at least 80% (w/w) of the total amount of emulsifier in the water-dispersible composition is the polysorbate emulsifier, more preferably that at least 90% (w/w) of the total amount of emulsifier in the water-dispersible composition is the polysorbate emulsifier, even more preferably that at least 97% (w/w) of the total amount of emulsifier in the water-dispersible composition is the polysorbate emulsifier and most preferably that essentially all of the total amount of emulsifier in the water-dispersible composition is the polysorbate emulsifier.

Preferably, the pH of the water-dispersible composition is a pH from 1 to 7, more preferably a pH from 2 to 5 and even more preferably a pH from 2.5 to 4.

Preferably, the water-dispersible composition as described herein also comprises an antioxidant (such as tocopherols, rosemary extract, ascorbic acid or ascorbates).

In one preferred embodiment the solid particles are being dispersed in the aqueous phase in the absence of a hydrocolloid.
The term "hydrocolloid" as used herein refers to a protective colloid which prevents agglomeration of the pigments and thus provides wetting and dispersing activity. Examples of hydrocolloids include gelatin, gum arabic, beet pectin and starch octenyl succinate derivatives.
Starch octenyl succinate is the common name given to starch n-octenyl succinate which is made by treating starch with n-octenyl succinic anhydride at pH 8-8.5. This type of starch derivative is anionic due to a carboxyl group and hydrophobic due to the C₈- alkene chain. The common E-number of starch sodium octenyl succinate derivatives is E1450 (see EU food additive legislation).

Preferably, the solid particles of the first aspect comprises carotenoid (preferably bixin and/or β-carotene) crystals covered with polysorbate and the solid particles comprise less than 0.5% (preferably 0%) (w/w) of fat, oil or wax.

In a particular preferred embodiment, the water-dispersible composition of the first aspect and herein relevant embodiments thereof is a water-dispersible composition, wherein the water-dispersible composition comprises a dispersion of at least 10% (w/w) of β-carotene as the natural pigment in the form of solid particles of an average size of at the most 10 µm, said particles being dispersed in an aqueous phase comprising polysorbate as the water-soluble emulsifier and wherein the ratio (w/w) of polysorbate emulsifier:β-carotene pigment is in the range of 1:3 to 3:1.
In relation to the embodiment immediately above - it is preferred that:
- the composition comprises at least 12% (w/w) of β-carotene, more preferably at least 14% (w/w) of β-carotene and even more preferably at least 18% (w/w) of β-carotene;
- the solid particles have an average size of at the most 4 µm, more preferably of at the most 2 µm, even more preferably of at the most 1.5 µm and most preferably of at the most 1.0 µm; and/or;
- the ratio (w/w) of polysorbate emulsifier:β-carotene pigment is in the range of 1:2 to 3:1, more preferably in the range of 1:2 to 2:1 and even more preferably in the range of 1:2 to 1.5:1; and/or
- wherein solid particles comprises β-carotene crystals covered with polysorbate and the solid particles comprise less than 0.5% (preferably 0%) (w/w) of fat, oil or wax.

In a particular preferred embodiment, the water-dispersible composition of the first aspect and herein described embodiments thereof is a water-dispersible composition, wherein the water-dispersible composition comprises a dispersion of at least 10% (w/w) of bixin as the natural pigment in the form of solid particles of an average size of at the most 10 µm, said particles being dispersed in an aqueous phase comprising polysorbate as the water-soluble emulsifier and wherein the ratio (w/w) of polysorbate emulsifier:bixin pigment is in the range of 1:3 to 3:1.
In relation to the embodiment immediately above - it is preferred that:
- the composition comprises at least 12% (w/w) of bixin, more preferably at least 14% (w/w) of bixin and even more preferably at least 18% (w/w) of bixin;
- the solid particles have an average size of at the most 4 µm, more preferably of at the most 2 µm, even more preferably of at the most 1.5 µm and most preferably of at the most 1.0 µm;
- the ratio (w/w) of polysorbate emulsifier:bixin pigment is in the range of 1:2 to 3:1, more preferably in the range of 1:2 to 2:1 and even more preferably in the range of 1:2 to 1.5:1; and/or
- wherein solid particles comprises bixin crystals covered with polysorbate and the solid particles comprise less than 0.5% (preferably 0%) (w/w) of fat, oil or wax.

The water-dispersible composition according to the present invention is useful in the manufacture of an edible product or a pharmaceutical product. An edible product may e.g. be a food product or a feed product.
The water-dispersible composition according to the present invention can be used as a colorant for food, feed and/or pharmaceutical products. The composition is particularly useful in low pH applications such as in application where pH of the product is below 7, such as below 6, such as below 4, or even below a pH of 3. Most food products are in the acidic range and a specific useful application includes coloring of beverage products. In beverage products the pH is typically around 2-3 and the beverage product is therefore preferably a beverage product, wherein the pH is from pH 2 to 3.

Preferred examples of a food product are beverage, wine gum, marmalade, jam, sugar confectionary, panned chocolate lentils, sausage casings, pasta, macaroni, cheese, prepared food or extruded foods.
Preferred example of a beverage is soft drink.

In a specific aspect the present invention relates to a pharmaceutical product comprising a composition according to the above description.

As discussed above - a fourth aspect of the invention relates to a method for preparing a water-dispersible pigment composition of the first aspect and herein relevant embodiments thereof, the method comprising preparing a dispersion of at least 2% (w/w) of carotenoid natural hydrophobic pigment in the form of solid particles by comminuting the pigment in an aqueous phase comprising polysorbate as a water-soluble emulsifier, to obtain a dispersion comprising the pigment in the form of solid particles of an average size of at the most 10 µm.

Preferably the comminuting is done by milling, preferably wet milling.
A preferred milling is ball milling.

As understood by the skilled person in the present context - preferred embodiments in relation to a water-dispersible composition of the first aspect may imply corresponding evident preferred embodiments in relation to the fourth aspect of the invention (relating to a method for preparing a water-dispersible pigment composition of the first aspect).

For instance, in relation to the method of the fourth aspect it may be preferred that:
- the pigment is comminuted in an aqueous phase in the absence of a hydrocolloid;
- the polysorbate emulsifier is present in an amount of at least 5% (w/w);
- the ratio of polysorbate emulsifier:carotenoid pigment is in a range of 1:10 to 4:1.

The invention will now be further illustrated in the following non-limiting examples.

### EXAMPLES

### Example 1: Composition with beta-carotene and polysorbate

**Composition of milled suspension:**

| Ingredient | % | Amount (g) |
|---|---|---|
| Dem. water | 73.8 | 2952 |
| Polysorbate 80 | 7.5 | 300 |
| Ascorbic acid | 3 | 120 |
| K-sorbate | 0,1 | 4 |
| Acetic acid | 0.6 | 24 |
| Beta-carotene crystals | 15 | 600 |
| Citric acid to pH 3.0 - 3.5 | | |

### Ball milling:

The color suspension was milled on a LabStar ball mill (Netzsch, Germany) in two steps.

The particle size of the milled beta-carotene crystals was measured on a Malvern Mastersizer.

During the first milling step the mill was equipped with 450 ml of beads (SiliBeads ZY, Sigmund Lindner GmbH, Germany) with diameters in the range 0.6-0.8 mm and a 0.2 mm slit on the mill.

Milling was continued until all particles with a diameter of above 10 microns had disappeared.

The beads were then exchanged with 450 ml of smaller beads (SiliBeads ZY, Sigmund Lindner GmbH, Germany) with diameters in the range of 0.3-0.4 mm.

Milling was continued until a particle size (d(4,3)) of 0.26 microns was obtained.
After storage for 3 months at 4 °C a mean particle size d(4,3) of 0.28 microns was measured.

The viscosity of the composition was still very low after milling and this was still the case after storage for 3 months at 4 °C

Beta-carotene strength was measured: 12.48%.

0.032 g of the color formulation was dissolved in 250 ml of a standard soft drink medium with a pH value of 3.0 and measured on a Minolta Chromameter, CT310.

Chroma was measured at 96.51, which is a high value.

### Example 2: Composition with crystalline bixin and polysorbate

**Composition of milled suspension:**

| Ingredient | % | Amount (g) |
|---|---|---|
| Dem. water | 73.8 | 2952 |
| Polysorbate 80 | 7.5 | 300 |
| Ascorbic acid | 3 | 120 |
| K-sorbate | 0.1 | 4 |
| Acetic acid | 0.6 | 24 |
| Bixin crystals 90% | 15 | 600 |
| Citric acid to pH 3.0 - 3.5 | | |

### Ball milling:

The color suspension was milled on a LabStar ball mill (Netzsch, Germany) in two steps.

The particle size of the milled bixin crystals was measured on a Malvern Mastersizer.

During the first milling step the mill was equipped with 450 ml of beads (SiliBeads ZY, Sigmund Lindner GmbH, Germany) with diameters in the range of 0.6-0.8 mm and a 0.2 mm slit on the mill.

Milling was continued until a particle size (d (4,3)) of 0.73 microns was obtained.

The beads were then exchanged with 450 ml of smaller beads (SiliBeads ZY, Sigmund Lindner GmbH, Germany) with diameters in the range of 0.3-0.4 mm and a 0.1 mm slit on the mill.

Milling was continued until a particle size (d (4,3)) of 0.28 microns was obtained.
After storage for 3 months at 4 °C a mean particle size d(4,3) of 0.28 microns was measured.

The viscosity of the composition was still very low after milling and this was still the case after storage for 3 months at 4 °C.

Bixin strength was measured: 13.0 %.

0.0385 g of the color formulation was dissolved in 250 ml of a standard soft drink medium with a pH value of 3.0 and measured on a Minolta Chromameter, CT310.

Chroma was measured at 90.18, which is a high value.

### Example 3: Composition with beta-carotene and polysorbate

**Composition of milled suspension:**

| Ingredient | % | Amount (g) |
|---|---|---|
| Dem. water | 66.3 | 2652 |
| Polysorbate 80 | 15 | 600 |
| Ascorbic acid | 3 | 120 |
| K-sorbate | 0.1 | 4 |
| Acetic acid | 0.6 | 24 |
| Beta-carotene crystals | 15 | 600 |
| Citric acid to pH 3.0 - 3.5 | | |

### Ball milling:

The color suspension was milled on a LabStar ball mill (Netzsch, Germany) in two steps.

The particle size of the milled beta-carotene crystals was measured on a Malvern Mastersizer.

During the first milling step the mill was equipped with 450 ml of beads (SiliBeads ZY, Sigmund Lindner GmbH, Germany) with diameters in the range 0.6-0.8 mm and a 0.2 mm slit on the mill.

Milling was continued until all particles with a diameter of above 10 microns had disappeared.

The beads were then exchanged with 450 ml of smaller beads (SiliBeads ZY, Sigmund Lindner GmbH, Germany) with diameters in the range of 0.3-0.4 mm.

Milling was continued until a particle size (d (4,3)) of less than 0.30 microns was obtained.

The viscosity of the composition was after milling still very low.

Beta-carotene strength was measured: around 12-13%.

### Example 4: Composition with crystalline bixin and polysorbate

| Ingredient | % | Amount (g) |
|---|---|---|
| Dem. Water | 63.3 | 2476 |
| Polysorbate 80 | 11 | 440 |
| Ascorbic acid | 4 | 160 |
| K-benzoate | 0,1 | 4 |
| Acetic acid 30 % | 0.6 | 80 |
| Bixin crystals 90 % | 21 | 840 |
| Citric acid to pH 3.0 - 3.5 | | |

### Ball milling:

The color suspension was milled on a LabStar ball mill (Netzsch, Germany) in two steps.

The particle size of the milled bixin crystals was measured on a Malvern Mastersizer.

During the first milling step the mill was equipped with 450 ml of beads (SiliBeads ZY, Sigmund Lindner GmbH, Germany) with diameters in the range of 0.6-0.8 mm and a 0.2 mm slit on the mill.
Milling was continued until a particle size (d (v,0.5)) of 0.42 microns was obtained.
The beads were then exchanged with 450 ml of smaller beads (SiliBeads ZY, Sigmund Lindner GmbH, Germany) with diameters in the range of 0.3-0.4 mm and a 0.1 mm slit on the mill.
Milling was continued until a particle size (d (v,0.5)) of 0.25 microns was obtained. After storage for 3 months at 4 °C a mean particle size d(v,0.5) of 0.28 microns was measured.

The viscosity of the composition was very low also after milling. This was still the case after storage for 3 months at 4 °C

### Example 5: Composition with beta-carotene and polysorbate

**Composition of milled suspension (Recipe 20 % beta-carotene):**

| Ingredient | % | Amount (g) |
|---|---|---|
| Dem water | 54.3 | 2118 |
| Polysorbate 80 | 20 | 800 |
| Ascorbic acid | 4 | 160 |
| K-sorbate | 0.1 | 4 |
| Acetic acid 30 % | 0.6 | 80 |
| Crystalline beta-carotene | 21 | 840 |
| Citric acid to pH 3.0 - 3.5 | | |

### Ball milling:

The color suspension was milled on a LabStar ball mill (Netzsch, Germany) in two steps. The particle size of the milled beta-carotene crystals was measured on a Malvern Mastersizer.

During the first milling step the mill was equipped with 450 ml of beads (SiliBeads ZY, Sigmund Lindner GmbH, Germany) with diameters in the range 0.6-0.8 mm and a 0.2 mm slit on the mill.
Milling was continued until all particles with a diameter of above 10 microns had disappeared.
The beads were then exchanged with 450 ml of smaller beads (SiliBeads ZY, Sigmund Lindner GmbH, Germany) with diameters in the range of 0.3-0.4 mm.

Milling was continued until a particle size (d (v,4,3)) of 0.28 microns was obtained.
After storage for 3 months at 4 °C a mean particle size d(v,4,3) of 0.29 microns was measured.

The viscosity of the composition was still very low after milling. This was still the case after storage for 3 months at 4 °C

### Example A: Composition with turmeric/curcumin powder and polysorbate (Comparative example)

**Composition of milled suspension:**

| Ingredient | % | Amount (g) |
|---|---|---|
| Dem. water | 68.2 | 2728 |
| Polysorbate 80 | 10 | 400 |
| K-sorbate | 0.1 | 4 |
| Acetic acid | 0.6 | 24 |
| Curcumin powder 95% | 21 | 840 |
| Citric acid to pH 3.0 - 3.5 | | |

### Ball milling:

The color suspension was milled on a LabStar ball mill (Netzsch, Germany) in two steps.

The particle size of the milled curcumin powder was measured on a Malvern Mastersizer.

During the first milling step the mill was equipped with 450 ml of beads (SiliBeads ZY, Sigmund Lindner GmbH, Germany) with a diameter in the range 0.6-0.8 mm and a 0.2 mm slit on the mill.

Milling was continued until a particle size (d (4,3)) of 0.47 microns was obtained.

The beads were then exchanged with 450 ml of smaller beads (SiliBeads ZY, Sigmund Lindner GmbH, Germany) with diameters in the range of 0.3-0.4 mm. and a 0.1 mm slit on the mill.

Milling was continued until a particle size (d(v,0.5) of 0.25 microns was obtained.
The viscosity of the composition was still very low.

After storage for two weeks at 4 °C in a common fridge viscosity increased dramatically and particle size also increased. D (v,0.5) in this period increased from 0.25 micron to 0.95 micron. This demonstrates that the combination of turmeric as the pigment and polysorbate does not work as it does not lead to stable coloring compositions.

### Example B: Composition with beta-carotene and citric acid esters of fatty acid mono/diglycerides (Comparative example)

**Composition of suspension:**

| Ingredient | % | Amount (g) |
|---|---|---|
| Dem. water | 79.9 | 3196 |
| Citric acid ester of fatty acid mono/diglycerides | | |
| (citrem N-12) | 10.0 | 400 |
| Ascorbic acid | 5.0 | 200 |
| K-sorbate | 0.1 | 4 |
| Acetic acid | 0.6 | 24 |
| Beta-carotene crystals | 5.0 | 200 |
| Citric acid to pH 3.0 - 3.5 | | |

### Ball milling:

It was attempted to mill the color suspension on a LabStar ball mill (Netzsch, Germany) equipped with 450 ml of beads (SiliBeads ZY, Sigmund Lindner GmbH, Germany) with diameters in the range 0.6-0.8 mm and a 0.2 mm slit on the mill.

This turned out to be impossible due to a very high viscosity.

### Example C: Composition with carbo vegetabilis and polysorbate (Comparative example)

**Composition of milled suspension (Recipe 5 % carbo):**

| Ingredient | % | Amount g |
|---|---|---|
| Dem water | 50 | 2000 |
| Acetic acid | 1 | 40 |
| Polysorbate | 15 | 600 |
| Carbo pasta 15 % carbo | 34 | 1360 |
| Citric acid to pH 3 | | |

### Ball milling:

The color suspension was milled on a LabStar ball mill (Netzsch, Germany) in two steps. The particle size of the milled beta-carotene crystals was measured on a Malvern Mastersizer.

During the first milling step the mill was equipped with 450 ml of beads (SiliBeads ZY, Sigmund Lindner GmbH, Germany) with diameters in the range 0.6-0.8 mm and a 0.2 mm slit on the mill.
Milling was continued until all particles with a diameter of above 10 microns had disappeared.
The beads were then exchanged with 450 ml of smaller beads (SiliBeads ZY, Sigmund Lindner GmbH, Germany) with diameters in the range of 0.3-0.4 mm.

Milling was continued until a particle size (d (v,0.5)) of 0.21 microns was obtained.
The viscosity of the composition was still very low
The milled composition was stored at 4 °C for 4 days. The particle size had then increased to d(v,0.5) 1.13 microns.
This demonstrates that this coloring composition is not stable.

### Example D: Composition with carbo vegetabilis and polysorbate (Comparative example)

**Composition of milled suspension (Recipe 5 % carbo):**

| Ingredient | % | Amount g |
|---|---|---|
| Dem water | 22.33 | 893.3 |
| Acetic acid | 1 | 40 |
| Polysorbate | 10 | 400 |
| Carbo pasta 15 % carbo | 66.7 | 2666.7 |
| Citric acid to pH 3 | | |

### Ball milling:

The color suspension was milled on a LabStar ball mill (Netzsch, Germany) in two steps. The particle size of the milled beta-carotene crystals was measured on a Malvern Mastersizer.

During the first milling step the mill was equipped with 450 ml of beads (SiliBeads ZY, Sigmund Lindner GmbH, Germany) with diameters in the range 0.6-0.8 mm and a 0.2 mm slit on the mill.
Milling was continued until all particles with a diameter of above 10 microns had disappeared.
The beads were then exchanged with 450 ml of smaller beads (SiliBeads ZY, Sigmund Lindner GmbH, Germany) with diameters in the range of 0.3-0.4 mm.

Milling was continued until a particle size (d (v,0.5)) of 0.20 microns was obtained.
The viscosity of the composition was still very low
The milled composition was stored at 4 °C for 4 days. The particle size had then increased to d(v,0.5) 0.90 microns.
This demonstrates that this coloring composition is not stable.

### Example E: Composition with copper chlorophyllin and polysorbate (Comparative Example)

Cu chlorophyllin /polysorbate: 2/1

**Water phase 1)**

| Ingredient | Grams |
|---|---|
| Dem. water | 1241 |
| Cu chlorophyllin | 520 |

Stirred until all Cu chlorophyllin has dissolved

**Water phase 2)**

| Ingredient | Grams |
|---|---|
| Dem. water | 1875 |
| K sorbate | 4 |
| H₂SO₄ conc. | 75 |

When all had dissolved water phase 1 was added to water phase 2 under stirring.
When all had been added the suspension was treated for 2 minutes on a Silverson.mixer to stir up lumps of precipitated Cu chlorophyllin
After this treatment pH was measured at 2.35.

260 g polysorbate was then added to the Cu chlorophyllin suspension.

pH measured at 2.43.

Stirred for two hours.

The suspension appeared very viscous, too viscous for ball milling.

pH raised by the addition of solid KOH.

After 2.33 g: pH: 2.66.

After another 7.15 g: pH: 4.06.

The precipitated suspension still appeared very viscous - too viscous for milling.

It was concluded that with this composition it will not be possible to produce a coloring composition according to the invention.

### Example F: Composition with copper chlorophyllin and polysorbate (Comparative example)

Cu chlorophyllin /polysorbate: 1/2

**Water phase 1)**

| Ingredient | Grams |
|---|---|
| Dem. water | 1131.9 |
| Cu chlorophyllin | 260 |

Stirred until all Cu chlorophyllin has dissolved

**Water phase 2) :**

| Ingredient | Grams |
|---|---|
| Dem. water | 937.5 |
| K sorbate | 2 |
| H₂SO₄ conc. | 37.5 |

When all had dissolved water phase 1 was added to water phase 2 under stirring.

pH was measured at 1.94 in the precipitated Cu chlorophyllin suspension.

540 g polysorbate was then added to the suspension.

When all had been added the suspension was treated for 5 minutes on a Silverson.

pH was then adjusted to 4.35 with solid KOH.

The suspension was stored overnight at 4 C for ball milling.

Viscosity had increased significantly overnight and the mill clogged.

The suspension was then diluted to a theoretical strength of 6 % and milled right after.

### Ball milling:

The color suspension was milled on a LabStar ball mill (Netzsch, Germany) in two steps. The particle size of the milled Cu chlorophyllin crystals was measured on a Malvern Mastersizer.

During the first milling step the mill was equipped with 450 ml of beads (SiliBeads ZY, Sigmund Lindner GmbH, Germany) with diameters in the range 0.6-0.8 mm and a 0.2 mm slit on the mill.
Milling was continued until all particles with a diameter of above 10 microns had disappeared.
The beads were then exchanged with 450 ml of smaller beads (SiliBeads ZY, Sigmund Lindner GmbH, Germany) with diameters in the range of 0.3-0.4 mm.

Milling was continued until a particle size (d (v,0.5)) of 0.13 microns was obtained.
The viscosity of the composition was still very low.
The milled composition was stored at 4 C for 4 days. The particle size had then increased to d(v,0.5) 0.44 microns.

It was concluded that this coloring composition was not stable.

## Claims

1. A water-dispersible composition comprising a dispersion of at least 2% (w/w) of carotenoid natural hydrophobic pigment in the form of solid particles of an average size of at the most 10 µm, said solid particles being dispersed in an aqueous phase comprising polysorbate as a water-soluble emulsifier and
wherein the ratio (w/w) of polysorbate emulsifier: carotenoid pigment is in the range of 1:10 to 4:1; and
wherein the carotenoid natural hydrophobic pigment is at least one pigment selected from the group consisting of: bixin, β-carotene, α-carotene, apocarotenals, canthaxanthin, saffron, crocin, capsanthin, capsorubin, lutein, astaxanthin, rubixanthin, violaxanthin, rhodoxanthin and lycopene.

2. The water-dispersible composition according to claim 1, wherein the carotenoid natural hydrophobic pigment is at least one pigment selected from the group consisting of: bixin and β-carotene.

3. The water-dispersible composition according to any of the preceding claims, wherein the ratio (w/w) of polysorbate emulsifier:carotenoid pigment is in the range of 1:4 to 3:1.

4. The water-dispersible composition according to any of the preceding claims, wherein the water-dispersible composition comprises a dispersion of at least 5% (w/w) of carotenoid natural hydrophobic pigment in the form of solid particles of an average size of at the most 10 µm.

5. The water-dispersible composition according to any of the preceding claims, wherein the polysorbate is polysorbate 20, polysorbate 40, polysorbate 60, polysorbate 65, polysorbate 80 or a mixture of these.

6. The water-dispersible composition according to any of the preceding claims, wherein the polysorbate water-soluble emulsifier is present in the composition in an amount of at least 5% (w/w).

7. The water-dispersible composition according to any of the preceding claims, wherein at least 80% (w/w) of the total amount of emulsifier in the water-dispersible composition is the polysorbate emulsifier.

8. The water-dispersible composition according to any of the preceding claims, wherein the pH of the water-dispersible composition is a pH from 2 to 5.

9. The water-dispersible composition according to any of the preceding claims, wherein the solid particles are being dispersed in the aqueous phase in the absence of a hydrocolloid.

10. The water-dispersible composition according to any of the preceding claims, wherein the solid particles of claim 1 comprise carotenoid crystals covered with polysorbate and the solid particles comprise less than 0.5% (w/w) of fat, oil or wax.

11. Use of a water-dispersible composition according to any of the preceding claims for the manufacture of an edible product or a pharmaceutical product.

12. The use according to claim 11, wherein said water-dispersible composition is used as a colorant for a food product, a feed product or a pharmaceutical product.

13. The use according to claim 12, wherein the food product is a beverage, wine gum, marmalade, jam, sugar confectionery, panned chocolate lentils, sausage casings, pasta, macaroni, cheese, prepared food or extruded foods.

14. An edible product comprising a water-dispersible composition according to any of the claims 1 to 10.

15. A method for preparing a water-dispersible pigment composition according to any of the claims 1 to 10, the method comprising preparing a dispersion of at least 2% (w/w) of carotenoid natural hydrophobic pigment in the form of solid particles by comminuting the pigment in an aqueous phase comprising polysorbate as a water-soluble emulsifier, to obtain a dispersion comprising the pigment in the form of solid particles of an average size of at the most 10 µm.

## Patentansprüche

1. Wasserdispergierbare Zusammensetzung, umfassend eine Dispersion von mindestens 2 % (Gew./Gew.) natürlichem hydrophobem Carotinoidpigment in Form von Feststoffteilchen einer durchschnittlichen Größe von höchstens 10 µm, wobei die Feststoffteilchen in einer wässrigen Phase dispergiert sind, die Polysorbat als einen wasserlöslichen Emulgator umfasst, und
wobei das Verhältnis (Gew./Gew.) von Polysorbat-Emulgator : Carotinoidpigment im Bereich von 1:10 bis 4:1 liegt; und
wobei das natürliche hydrophobe Carotinoidpigment mindestens ein Pigment ist, das aus der Gruppe ausgewählt ist, die aus Folgendem besteht: Bixin, β-Carotin, α-Carotin, Apocarotinale, Canthaxanthin, Safran, Crocin, Capsanthin, Capsorubin, Lutein, Astaxanthin, Rubixanthin, Violaxanthin, Rhodoxanthin und Lycopin.

2. Wasserdispergierbare Zusammensetzung nach Anspruch 1, wobei das natürliche hydrophobe Carotinoidpigment mindestens ein Pigment ist, das aus der Gruppe ausgewählt ist, die aus Folgendem besteht: Bixin und β-Carotin.

3. Wasserdispergierbare Zusammensetzung nach einem der vorstehenden Ansprüche, wobei das Verhältnis (Gew./Gew.) von Polysorbat-Emulgator : Carotinoidpigment im Bereich von 1:4 bis 3:1 liegt.

4. Wasserdispergierbare Zusammensetzung nach einem der vorstehenden Ansprüche, wobei die wasserdispergierbare Zusammensetzung eine Dispersion von mindestens 5 % (Gew./Gew.) natürlichem hydrophobem Carotinoidpigment in Form von Feststoffteilchen einer durchschnittlichen Größe von höchstens 10 µm umfasst.

5. Wasserdispergierbare Zusammensetzung nach einem der vorstehenden Ansprüche, wobei das Polysorbat Polysorbat 20, Polysorbat 40, Polysorbat 60, Polysorbat 65, Polysorbat 80 oder eine Mischung davon ist.

6. Wasserdispergierbare Zusammensetzung nach einem der vorstehenden Ansprüche, wobei der wasserlösliche Polysorbat-Emulgator in der Zusammensetzung in einer Menge von mindestens 5 % (Gew./Gew.) vorhanden ist.

7. Wasserdispergierbare Zusammensetzung nach einem der vorstehenden Ansprüche, wobei mindestens 80 % (Gew./Gew.) der Gesamtemulgatormenge in der wasserdispergierbaren Zusammensetzung der Polysorbat-Emulgator ausmacht.

8. Wasserdispergierbare Zusammensetzung nach einem der vorstehenden Ansprüche, wobei der pH-Wert der wasserdispergierbaren Zusammensetzung ein pH-Wert von 2 bis 5 ist.

9. Wasserdispergierbare Zusammensetzung nach einem der vorstehenden Ansprüche, wobei die Feststoffteilchen in der wässrigen Phase in Abwesenheit eines Hydrokolloids dispergiert werden.

10. Wasserdispergierbare Zusammensetzung nach einem der vorstehenden Ansprüche, wobei die Feststoffteilchen nach Anspruch 1 Carotinoidkristalle umfassen, die mit Polysorbat bedeckt sind, und die Feststoffteilchen weniger als 0,5 % (Gew./Gew.) Fett, Öl oder Wachs umfassen.

11. Verwendung einer wasserdispergierbaren Zusammensetzung nach einem der vorstehenden Ansprüche zur Herstellung eines essbaren Produktes oder eines pharmazeutischen Produktes.

12. Verwendung nach Anspruch 11, wobei die wasserdispergierbare Zusammensetzung als ein Färbemittel für ein Nahrungsmittelprodukt, ein Futtermittelprodukt oder ein pharmazeutisches Produkt verwendet wird.

13. Verwendung nach Anspruch 12, wobei das Nahrungsmittelprodukt ein Getränk, ein Weingummi, eine Marmelade, Konfitüre, Zuckersüßwaren, dragierte Schokoladenlinsen, Wursthüllen, Teigwaren, Makkaroni, Käse, Fertiggerichte oder extrudierte Lebensmittel ist.

14. Essbares Produkt, umfassend eine wasserdispergierbare Zusammensetzung nach einem der Ansprüche 1 bis 10.

15. Verfahren zum Herstellen einer wasserdispergierbaren Pigmentzusammensetzung nach einem der Ansprüche 1 bis 10, wobei das Verfahren das Herstellen einer Dispersion von mindestens 2 % (Gew./Gew.) natürlichem hydrophobem Carotinoidpigment in Form von Feststoffteilchen durch Zerkleinern des Pigments in einer wässrigen Phase umfasst, die Polysorbat als einen wasserlöslichen Emulgator umfasst, um eine Dispersion zu erhalten, die das Pigment in Form von Feststoffteilchen einer durchschnittlichen Größe von höchstens 10 µm umfasst.

## Revendications

1. Composition dispersible dans l'eau comprenant une dispersion d'au moins 2 % (p/p) de pigment hydrophobe naturel de caroténoïde sous la forme de particules solides d'une taille moyenne d'au plus 10 µm, lesdites particules solides étant dispersées dans une phase aqueuse comprenant du polysorbate en tant qu'émulsifiant soluble dans l'eau et
dans laquelle le rapport (p/p) émulsifiant polysorbate :
pigment de caroténoïde est compris dans l'intervalle de 1:10 à 4:1 ; et
dans laquelle le pigment hydrophobe naturel de caroténoïde est au moins un pigment choisi dans le groupe constitué de : bixine, β-carotène, α-carotène, apocaroténaux, canthaxanthine, safran, crocine, capsanthine, lutéine, astaxanthine, rubaxanthine, violoxanthantine, ranthoxanthine et lycopène.

2. Composition dispersible dans l'eau selon la revendication 1, dans laquelle le pigment hydrophobe naturel de caroténoïde est au moins un pigment choisi dans le groupe constitué de : bixine et β-carotène.

3. Composition dispersible dans l'eau selon l'une quelconque des revendications précédentes, dans laquelle le rapport (p/p) émulsifiant polysorbate : pigment de caroténoïde est compris dans l'intervalle de 1:4 à 3:1.

4. Composition dispersible dans l'eau selon l'une quelconque des revendications précédentes, dans laquelle la composition dispersible dans l'eau comprend une dispersion d'au moins 5 % (p/p) de pigment hydrophobe naturel de caroténoïde sous la forme de particules solides d'une taille moyenne d'au plus 10 µm.

5. Composition dispersible dans l'eau selon l'une quelconque des revendications précédentes, dans laquelle le polysorbate est le polysorbate 20, le polysorbate 40, le polysorbate 60, le polysorbate 65, le polysorbate 80 ou un mélange de ceux-ci.

6. Composition dispersible dans l'eau selon l'une quelconque des revendications précédentes, dans laquelle l'émulsifiant polysorbate soluble dans l'eau est présent dans la composition en une quantité d'au moins 5 % (p/p).

7. Composition dispersible dans l'eau selon l'une quelconque des revendications précédentes, dans laquelle au moins 80 % (p/p) de la quantité totale d'émulsifiant dans la composition dispersible dans l'eau est l'émulsifiant polysorbate.

8. Composition dispersible dans l'eau selon l'une quelconque des revendications précédentes, dans laquelle le pH de la composition dispersible dans l'eau est un pH de 2 à 5.

9. Composition dispersible dans l'eau selon l'une quelconque des revendications précédentes, dans laquelle les particules solides sont dispersées dans la phase aqueuse en l'absence d'un hydrocolloïde.

10. Composition dispersible dans l'eau selon l'une quelconque des revendications précédentes, dans laquelle les particules solides selon la revendication 1 comprennent des cristaux de caroténoïde recouverts de polysorbate et les particules solides comprennent moins de 0,5 % (p/p) de graisse, d'huile ou de cire.

11. Utilisation d'une composition dispersible dans l'eau selon l'une quelconque des revendications précédentes pour la fabrication d'un produit comestible ou d'un produit pharmaceutique.

12. Utilisation selon la revendication 11, dans laquelle ladite composition dispersible dans l'eau est utilisée en tant que colorant pour un produit alimentaire, un produit alimentaire pour animaux ou un produit pharmaceutique.

13. Utilisation selon la revendication 12, dans laquelle le produit alimentaire est une boisson, de la gomme au vin, de la marmelade, de la confiture, des sucreries, des dragées dures au chocolat, des boyaux à saucisse, des pâtes, des macaronis, du fromage, des plats préparés ou des aliments extrudés.

14. Produit comestible comprenant une composition dispersible dans l'eau selon l'une quelconque des revendications 1 à 10.

15. Procédé de préparation d'une composition de pigment dispersible dans l'eau selon l'une quelconque des revendications 1 à 10, le procédé comprenant la préparation d'une dispersion d'au moins 2 % (p/p) de pigment hydrophobe naturel de caroténoïde sous la forme de particules solides en fragmentant le pigment en une phase aqueuse comprenant du polysorbate en tant qu'émulsifiant soluble dans l'eau, pour obtenir une dispersion comprenant le pigment sous forme de particules solides d'une taille moyenne d'au plus 10 µm.
